# EUROPEAN PATENT SPECIFICATION

(11) **EP 1 923 087 B1**
(45) Date of publication and mention of the grant of the patent: **05.09.2012**
(21) Application number: 08075104.3
(22) Date of filing: 21.07.2000
(51) Int. Cl.: A61M 15/00

(54) **Unit dose capsules for dry powder inhaler**
Einheitsdosenkapseln für Trockenpulverinhalator
Capsules à dose d'unité pour inhalateur de poudre sèche

(30) Priority: 23.07.1999 US 145464 P; 22.05.2000 US 206123 P
(43) Date of publication of application: 21.05.2008
(62) Divisional of application: 05077995.8
(73) Proprietor: MannKind Corporation, Valencia, CA 91355 (US)
(72) Inventor: Steiner, Solomon S., Mount Kisco, NY 10507 (US); Poole, Trent, South Amherst, MA 01002 (US); Feldstein, Robert, Yonkers, NY 10701 (US); Fog, Per B., Bedford Hills, NY 10507 (US)
(74) Representative: Powell, Timothy John

(56) References cited:
- EP-A- 0 395 291
- WO-A-96/01105
- WO-A-98/39043
- DE-A1- 19 637 125
- US-A- 4 487 327
- US-A- 5 170 801
- US-A- 5 476 093

## Description

### FIELD OF THE INVENTION

The present invention is in the field of inhalers.

### BACKGROUND OF THE INVENTION

In the early 1970's it was found that certain medicines could be administered in drypowder form directly to the lungs by inhalation through the mouth or inspiration through the nose. This process allows the medicine to bypass the digestive system, and may, in certain cases, allow smaller does to be used to achieve the same results or orally ingested or injected medicines. In some cases, it provides a delivery technique that reduces side effects for medicines taken by other medicines.

Inhaler devices typically deliver their medicinal in a liquid mist or a powder mist. The liquid mist is typically created by a chlorofluorocarbon propellant. However, with the ban on chlorofluorocarbons by the Montreal protocol, interest has turned to dry powder inhalers.

For a dry powder inhaler to work effectively, it must deliver fine particles of medicinal powder that do not agglomerate, and do not end up striking, and being absorbed by the patient's mouth or upper oropharyngeal region. Air flow must therefore not be too fast. Furthermore, it should not be difficult for a patient to load with medicine or to use with the proper technique. Current dry particle inhalers fail in one or more of these important criteria.

European Patent Application EP 0 395 291 describes a container for additive materials for smoking articles, the container comprising two interconnected tubular elements, wherein there are openings, provided in the walls of the two elements.

### SUMMARY OF THE INVENTION

In its broadest aspect the invention is as defined in Claim 1.

Described is a dry powder inhaler comprising an intake section; a mixing section, and a mouthpiece. The mouthpiece is connected by a swivel joint to the mixing section, and may swivel back onto the intake section and be enclosed by a cover. The intake chamber comprises a special piston with a tapered piston rod and spring, and one or more bleed-through orifices to modulate the flow of air through the device. The intake chamber further optionally comprises a feedback module to generate a tone indicating to the user when the proper rate of airflow has been achieved. The mixing section holds a capsule with holes containing a dry powder medicament, and the cover only can open when the mouthpiece is at a certain angle to the intake section. The mixing section further opens and closes the capsule when the intake section is at a certain angle to the mouthpiece. The mixing section is a Venturi chamber configured by protrusions or spirals to impart a cyclonic flow to air passing through the mixing chamber. The mouthpiece includes a tongue depressor, and a protrusion to contact the lips of the user to tell the user that the DPI is in the correct position. An optional storage section, with a cover, holds additional capsules. The cover for the mouthpiece, and the cover for the storage section may both be transparent magnifying lenses.

The capsules may be two-part capsules where each portion has apertures which correspond to apertures in the other half when each half is partially fitted to the other half, and fully fitted to the other half. All the apertures may be closed when the two halves are rotated around their longitudinal axes with respect to each other. Each capsule may have a unique key on each half that only fits with a particular inhaler.

### BRIEF DESCRIPTION OF THE SEVERAL VIEWS OF THE DRAWINGS

Figure 1 is a schematic view of the dry particle inhaler described herein.
Figure 2 is schematic view of the mouthpiece cover.
Figure 3 is schematic view showing the angle between the intake section and the mouthpiece.
Figure 4 is a schematic view of the dry particle inhaler, showing the storage section.
Figure 5 is a schematic view of the intake section of the dry particle inhaler, showing the flow regulator and the feedback module.
Figure 6 is a schematic view of the mixing section.
Figure 7 is a schematic view of a capsule to hold medicament.
Figure 8 is a schematic view of the mouthpiece.
Figure 9 is a perspective view of a specific example of the dry particle inhaler in the closed position, with a capsule inserted into the mixing section, and extra capsules stored in the storage section.
Figure 10 is a perspective view of a specific example of the dry particle inhaler showing a capsule being loaded in to the mixing section.
Figure 11 is a perspective view of a specific example of the dry particle inhaler showing a capsule inserted into the mixing section, and the mouthpiece extended for use.
Figures 12,13,14, and 15 follow each other in temporal sequence.

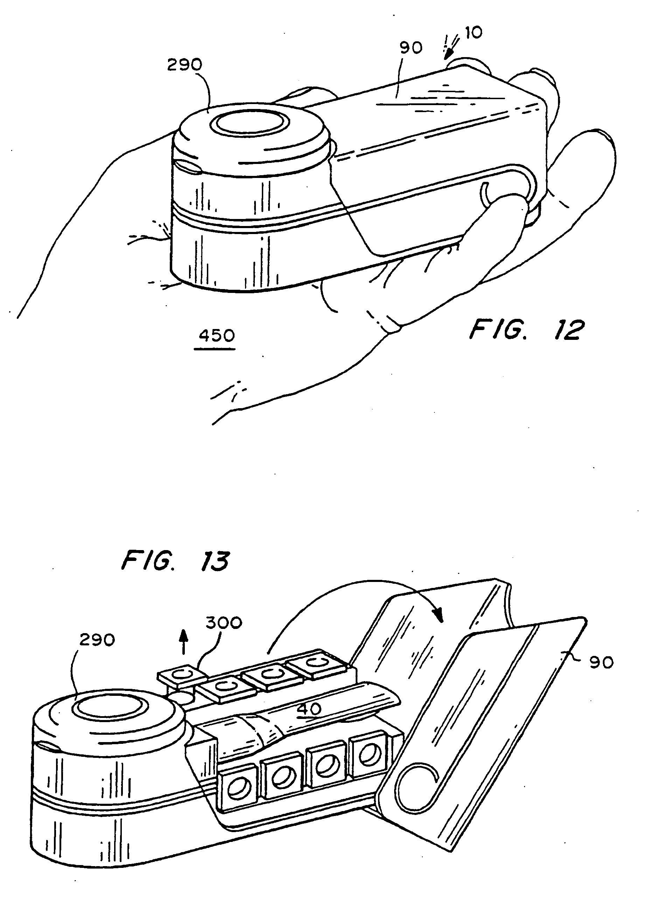

Figure 12 is a perspective view of a specific example of the dry particle inhaler showing a closed mouthpiece cover.
Figure 13 is a perspective view of a specific example of the dry particle inhaler showing an open mouthpiece cover.

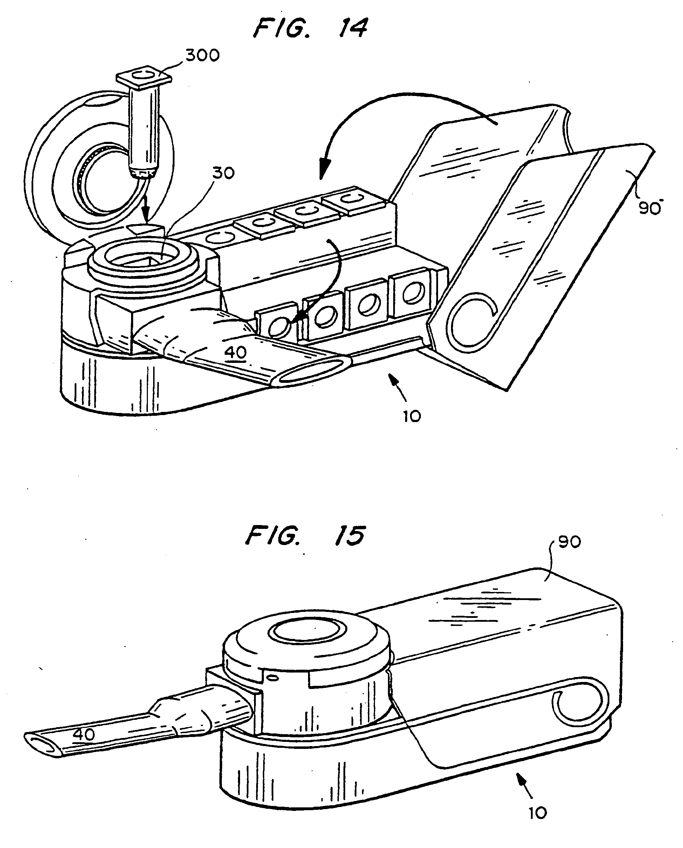

Figure 14 is a perspective view of a specific example of the dry particle inhaler showing an open mouthpiece cover, an open mixing section cover, and a capsule about to be inserted into the mixing section.
Figure 15 is a perspective view of a specific example of the dry particle inhaler showing the mouthpiece extended for use.

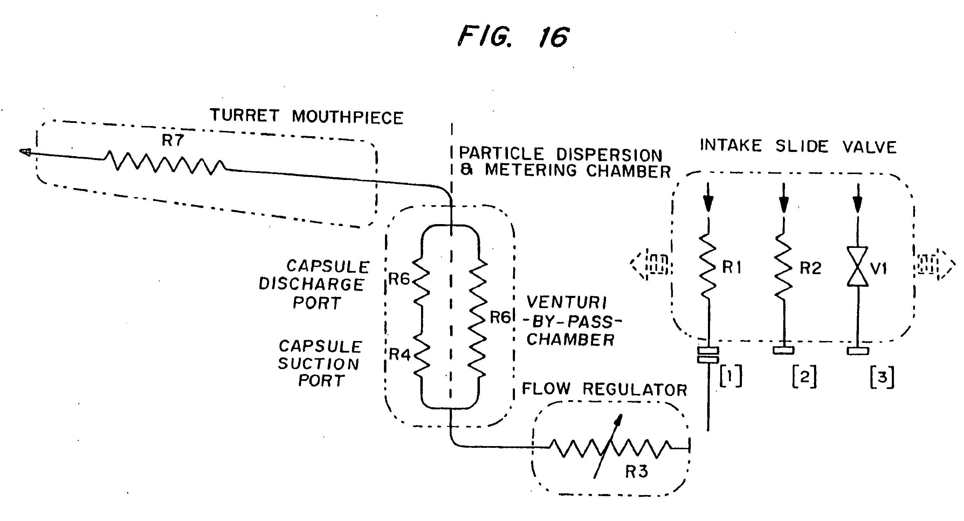

Figure 16 is a view of a pneumatic circuit, where air flows (fluid flows) are represented by their electrical equivalents.

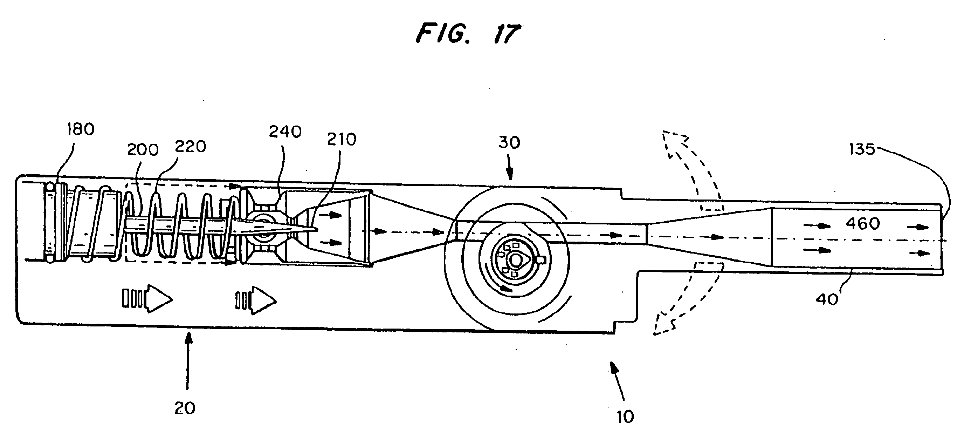

Figure 17 is a schematic view of the dry particle inhaler.

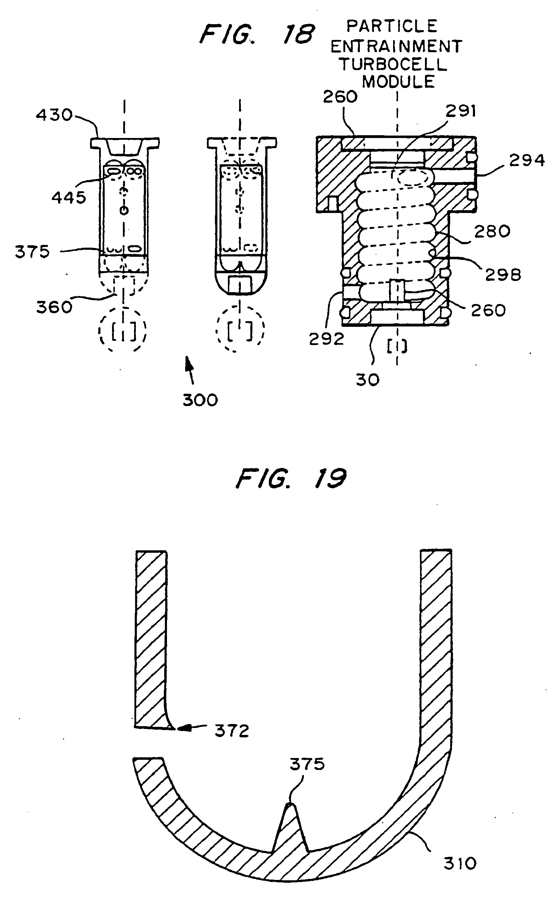

Figure 18 is a cutaway view of a capsule and a portion of the mixing section.
Figure 19 is a cutaway view of half of a capsule, showing a cone in the interior and a secondary hole with a chamfered, or beveled, edge.

### TABLE OF REFERENCE NUMBERS

- 10: dry powder inhaler device
- 20: intake section
- 30: mixing section
- 40: mouthpiece
- 50: air passage through dry powder inhaler device
- 60: longitudinal axis of intake section
- 70: longitudinal axis of mouthpiece section
- 80: swivel joint connecting mouthpiece and mixing section
- 90: cover for mouthpiece
- 100: protrusions on mouthpiece cover
- 110: depressions on dry particle inhaler cover to mate with protrusions on mouthpiece cover
- 120: tongue depressor on mouthpiece
- 130: protrusion on surface of mouthpiece to contact lips of device user
- 135: opening of mouthpiece to be fitted into user's mouth
- 140: intake port
- 150: flow regulator
- 160: bleed orifice
- 170: piston
- 180: piston head
- 190: piston rod
- 200: proximal portion of piston rod
- 210: distal portion of piston rod
- 220: spring
- 230: inner walls of intake section inner chamber
- 240: feedback module
- 250: mechanical fasteners in storage section
- 260: holder in mixing section for capsule
- 270: Venturi chamber
- 280: spiral shape or protrusions to impart cyclonic flow to air
- 290: cover for mixing chamber
- 291: interior of mixing section
- 292: air flow entrance to mixing section
- 294: air flow exit from mixing section
- 296: latch mechanism for mixing section cover
- 298: interior wall of mixing section
- 300: capsule
- 310: first tube
- 320: open end of first tube
- 330: closed end of first tube
- 340: long axis of first tube
- 350: protrusion on first tube
- 360: keying surface on first tube
- 370: secondary holes in first tube
- 372: chamfered edge of secondary hole
- 375: cone in interior of first tube
- 380: second tube
- 390: open end of second tube
- 400: closed end of second tube
- 410: long axis of second tube
- 420: protrusion on second tube
- 430: keying surface on second tube
- 440: secondary holes in second tube
- 445: cone in interior of second tube
- 450: hand of user
- 460: air flow direction
- 470: storage section
- 480: storage section cover

### DETAILED DESCRIPTION OF THE INVENTION

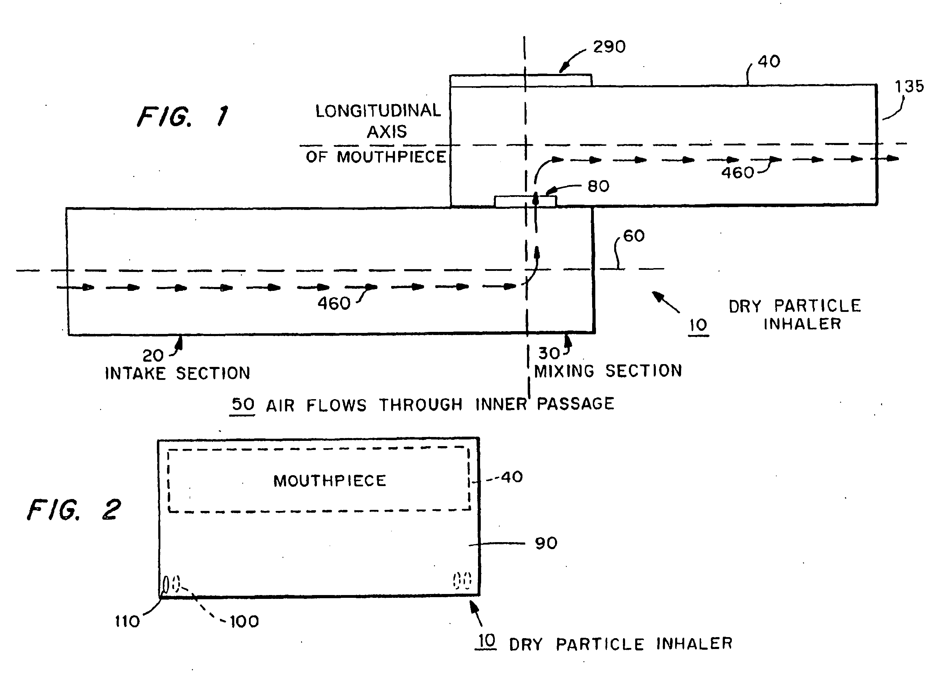

Figure 1 is a schematic drawing of the dry powder inhaler (10) described herein. It comprises an intake section (20), a mixing section (30) and a mouthpiece (40). An air passage (50) goes through the intake section (20), a mixing section (30) and a mouthpiece (40). A swivel joint (80) connects the mouthpiece (40) to the mixing section (30). The mixing section (20) has a cover (290) which may be a transparent magnifying lens. Arrow (460) shows the direction of air flow through the air passage (50) through the dry powder inhaler (10).
Figure 2 shows the mouthpiece cover (90) in the closed position over the dry particle inhaler (10). Protrusions (100) on the mouthpiece cover (90) mate with grooves or depressions (110) on the dry particle inhaler (10), to join the mouthpiece cover (90) to the dry particle inhaler (10).

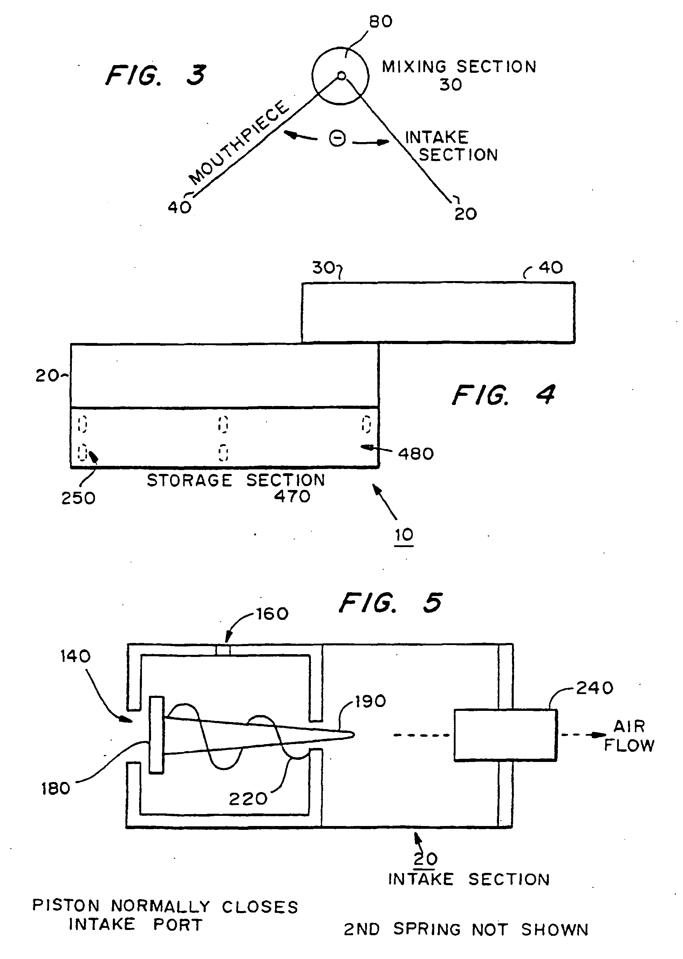

Figure 3 is a schematic of the DPI showing the mouthpiece (40) and the intake section (20) as represented by the longitudinal axis of the mouthpiece (70) and the longitudinal axis of the intake section (60). The swivel joint (80) connecting the mouthpiece (40) to the intake section (20) at the mixing section (30) may be regarded as the vertex of the angle. The importance of the angle (here called theta) between these two longitudinal axes will be further explained.
Figure 4 shows the dry particle inhaler (10) with a storage section (470). Indicated as being inside the storage section (470) are mechanical fasteners (250) which operate to hold medicament capsules (300) (not shown in this Figure) in the storage section. In this embodiment, the storage section (470) is shown as appended to the intake section (20). The storage section has a cover (480) which may be a transparent magnifying lens, to allow the user to easily read writing on medicament capsules stored therein. The storage section cover (480) may swivel outward, or slide open on a track (not shown), or open by a variety of mechanisms known to those of skill in the art.
Figure 5 shows the intake section (20) of the dry particle inhaler (10). The direction of air flow is shown by the arrow (460). Air is admitted through an intake port (140) and one or more bleed orifices (160) [The bleed orifices may also be styled as secondary ambient air intake ports]. The piston (170) normally covers the intake port (140). When the user (not shown) inspires, the piston head (180) is drawn backwards, at a steady rate modulated by the spring (220). The spring (220) is fixed to the piston (170) and the inner wall (230) of the intake section chamber. Thus the rate of air flow is controlled. The air flow is further controlled by the tapering of the piston rod (190), past which the air flows. For further control of the air flow, a second spring (not shown) may also control the rate of movement of the piston (170).

The piston (170) and spring (220) combination allow the user (not shown) to generate a vacuum in his lungs before the intake port (140) opens. Thus, by the time enough vacuum is generated to open the intake port (140), there will be sufficient air flow at a sufficient rate in the dry particle inhaler (10) to draw most of the medicament in the capsule (not shown) out of the inhaler into the proper place in the lungs of the user.

A feedback module (240) generates a signal to the user (not shown), which tells the user whether he is inspiring at the correct rate. The signal may be an audible one, in one embodiment a tone that is at a steady pitch when air flow is at a certain steady rate. In one embodiment of the dry particle inhaler (10), the signal is generated mechanically, such as by a musical reed. In another example, the signal might be generated electronically, after electronic measurement of the air flow rate. The feedback module (240) would include a means for increasing or lessening the signal strength, or turning the signal off entirely. If the signal were generated by a reed, the mechanism for turning off the signal might be covering a bleed orifice which might admit the air flow generating the signal. If the signal were generated electronically, a simple push button or dial might turn on and off the signal.

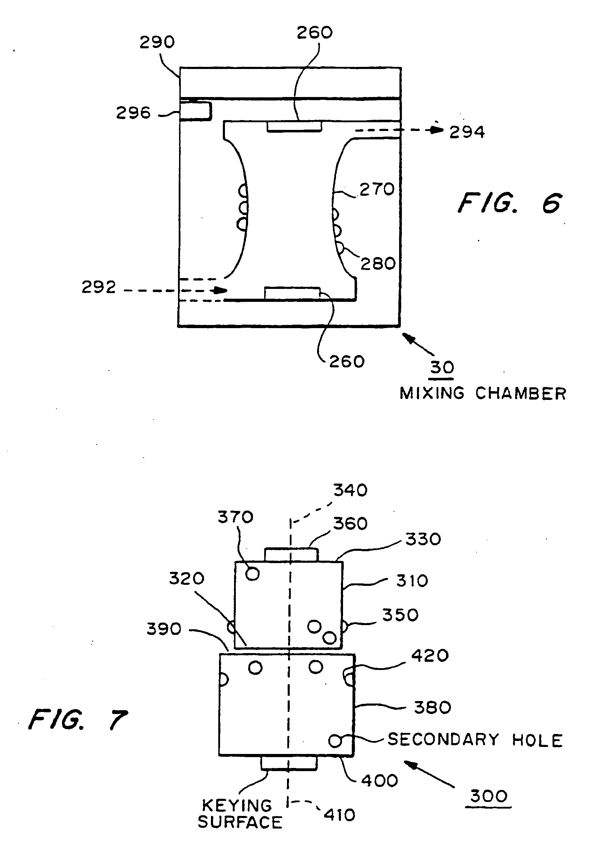

Figure 6 shows a schematic of the mixing section (30) of the present invention. The mixing section has a cover (290), and a holder (260) for a medicament capsule (not shown). The holder (260) is a mechanism which grips and turns the capsule (not shown) to open and close it as the longitudinal axis (70) of the mouthpiece is rotated about the swivel joint (80) relative to the longitudinal axis (60) of the intake section. Such a mechanism may be straightforward: in a simplest embodiment, both the top and bottom halves (not shown) of the capsule could be fixed to their respective holders (260).

The Venturi chamber (270) speeds the flow of air near the capsule (not shown). Air flows in at (292), and out through (294). In one embodiment, air flows both through and around a capsule (not shown) holding a dry powder medicament. The special shape of the Venturi chamber (270), which further includes protrusions or spiral shapes (280), imparts a cyclonic flow to the air passing through the mixing section (30). This helps to deagglomerate particles of dry powder. The spiral shape of the interior of the mixing section (291) can be two separate spirals, in one embodiment of the invention. Mixing section (30) therefore provides the means whereby air flow is speeded up to suspend dry particles in air and de- agglomerate them, and then slow the air flow somewhat while the particles are still suspended in air. The cover (290) for the mixing section (30) may be a transparent magnifying lens, so that any writing on the capsule (not shown) may be read easily.

In one example of the dry particle inhaler (10), the cover (290) of the mixing section may not be opened unless the longitudinal axis (70) of the mouthpiece forms a certain angle with the longitudinal axis (60) of the intake section, with the vertex of the angle being the swivel joint (80) connecting the mouthpiece (40) and the mixing section (30). The latch mechanism (296) for the cover (290) of the mixing section can accomplish this, by any of several mechanical means known to those of ordinary skill in the art. In the simplest embodiment, a catchment (not shown) in the cover (290) for the mixing chamber would be engaged by a slip ring (not shown) on the mixing section which was only a certain number of degrees of a circle. If the mouthpiece (40) is rotated enough relative to the intake section (20), the slip ring (not shown) would no longer engage the catchment (not shown). In one example, the user could open the cover (290) if the angle is between approximately ninety and one-hundred and eighty degrees.

Figure 7 shows a medicament capsule (300) for use with an inhaler, be it a dry powder inhaler (10), or a liquid mist inhaler. The capsule (300) has two halves which fit together, here styled a first tube (310) and a second tube (380). Each tube has an open end (320,390), and a closed end (330,400). Each tube also has a long axis (340,410). In addition, each tube has a number of secondary holes (370,440). The first tube (310) fits inside the second tube (380) snugly. A protrusion (350) on the outer surface of the first tube (310) can slide past a corresponding protrusion (420) on the inner surface of the second tube (380). This locks the first tube (310) to the second tube (380). Therefore the first tube (310) and the second tube (380) have both an unlocked and a locked position. In the unlocked position, at least one secondary hole (370) in the first tube aligns with at least one secondary hole (440) in the second tube. This permits introduction of a medicament (not shown) into the capsule through the aligned secondary holes (370,440). The first tube (310) may then be locked to the second tube (380). When a user (not shown) is ready to use a capsule (300), he simply places it in the holder (260) in the mixing section (30), and closes the cover (290). When the holder (260) rotates the first tube (310) around its long axis (340) relative to the second tube (380) and its long axis (410) (the axes are now coincident), that causes at least two secondary holes (370) in the first tube to align with at least two secondary holes (440) in the second tube. Air can now pass in, through, and out of the capsule (300), releasing the medicament contained therein. In one embodiment of the inhaler, the capsule (300) might open when the angle between the longitudinal axis (70) of the mouthpiece section, the vertex of the swivel joint (80), and the longitudinal axis (70) of the mouthpiece section were between one hundred and seventy and one- hundred and eighty degrees. This rotation of the mouthpiece (40) relative to the intake section (20) would cause a corresponding rotation of the first tube (310) about its long axis (340) relative to the second tube (380) and its long axis (410).

In one embodiment of the invention, several protrusions on the surfaces of the first tube or the second tube might provide a variety of locking positions. Similarly, a variety of secondary holes in the first and second tubes might provide a variety of rotational positions aligning or not aligning secondary holes on the first and second tubes.

The capsules described herein permit the introduction of liquid or gel medicament which can be dried in the capsule, creating a powder. This permits the accurate production of very small amounts of powdered medicament in a capsule, since it can be formed from a larger volume of accurately metered liquid or gel medicament. This permits very accurate microdosing. In addition, chemical reactions and drug mixtures may be made directly in the capsules described herein, then the resulting formulation dried.

In one embodiment of the capsule (300), one or more of the secondary holes (370,440) used to admit air to the capsule is oval-shaped (elliptical). In one embodiment of the invention, the ratio of the long axis of the ellipse to the shorter axis may be between 1: 1 and 3: 1, and may be 2: 1. This ratio may be called a vertical aspect ratio. In one embodiment of the invention, the intersection of the surface defining one or more of the secondary holes (370,440) and the surface defining the interior of the capsule (300) meet in a chamfered, or beveled, edge. This chamfered edge creates a vortex when air flows through the secondary holes (370,440).

Each capsule (300) also has a keying surface (or fastening mechanism) on the closed end (330) of the first tube and the closed end (400) of the second tube comprising the capsule. The keying surface (360) on the first tube may be different from the keying surface (430) on the second tube. That permits easy tactile and visual identification of the orientation of the capsule. It also permits a system where each drug formulation in a capsule (300) corresponds to a dry particle inhaler (10), so users cannot mix up drugs. In one embodiment of the invention, the keying surface (360) of the first tube mates with a keying surface (430) of a different second tube, or the mechanical fasteners (250) of the storage section (470). This permits easy storage of the capsules (300) in the storage section (470).

Figure 18 shows a medicament capsule (300), with a keying surface (360) on the first tube and a keying surface (430) on the second tube. It also shows a cutaway view of the mixing section (30) and the air flow entrance (292) to the mixing section and the air flow exit (294) to the mixing section. A spiral shape (280) is given to the interior walls (298) of the mixing section, to impart a cyclonic flow to air passing through. The air flow entrance (292) and air flow exit (294) in this embodiment are tangential to the imaginary tube we might call the mixing section interior (291). That is to say, if a radius were drawn perpendicular to the long axis of the tube, and a tangent line were drawn to the circle perpendicular to the radius, the air flow would exit the mixing section along that tangent line. The tangential air flow exit (294) increases the velocity of the air flow, and thus helps disperse the medicament particles. As can be seen from Figure 18, the mixing section interior (291) is sized to accommodate a medicament capsule (300). Keying mechanisms (360,430) are shaped to mate with holder (260) in the mixing section. Capsules according to the present invention may have a number of shapes, including ovoid and rectangular shapes. A variety of shapes of protrusions and slots may also be employed as keying surfaces. For instance, a keying surface might be a rectangular block, and a capsule holder might have a rectangular orifice. Alternatively, a keying surface might be triangular, hexagonal, Z-shaped, C-shaped, etc., and the holder would have the correspondingly shaped aperture.

Figure 18 also shows one embodiment of the capsule (300) where a cone (375) is located in the interior of the first tube, and a cone (445) is located in the interior of the second tube. These cones (375,445) cause the air flow within the capsule to be cyclonic, aiding in mixing the medicament particles with the air. A cone is shown herein, but other cyclone-creating structures are contemplated by the present invention.

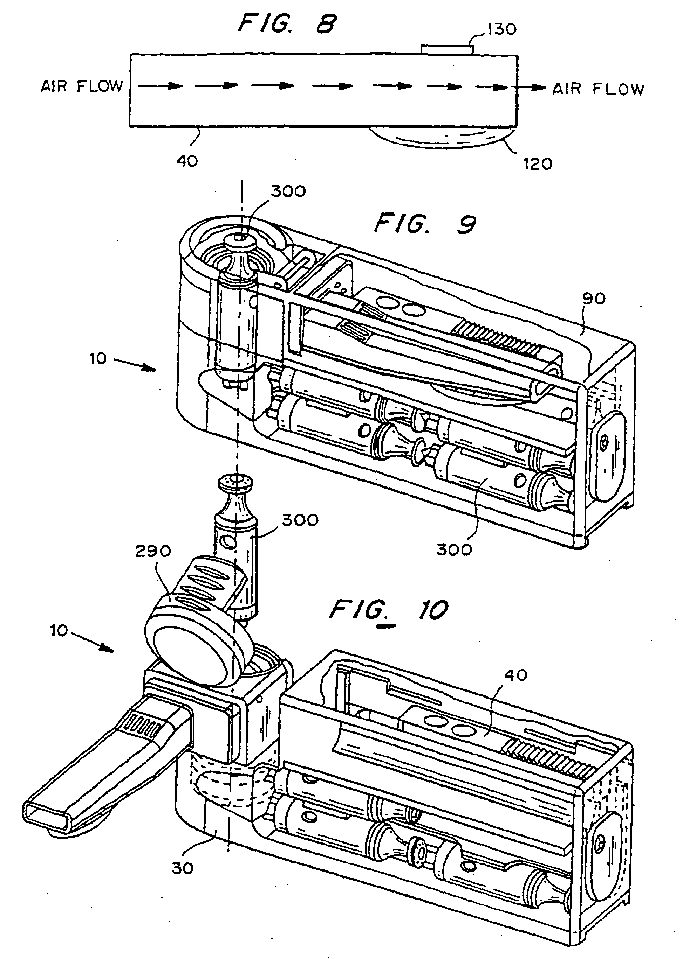

Figure 8 shows the mouthpiece (40) of the dry particle inhaler (10). It has a protrusion (130) on its surface to contact the lips of a user (not shown). This helps the user place the mouthpiece correctly in his mouth. The mouthpiece (40) also includes a tongue depressor (120), which may have a bulbous shape. The mouthpiece (40) is long enough that it fits approximately midway into the user's mouth (not shown). This permits greater delivery of medicament to the lungs, and less delivery to the oral cavity. The mouthpiece (40) has a particular aspect ratio of its inner channel (50) (see Figure 17). This slows the air passing through the channel so that the air borne particulates do not end up striking the back of the user's throat. However, the air is not slowed so much that the particulates settle out of the air flow.

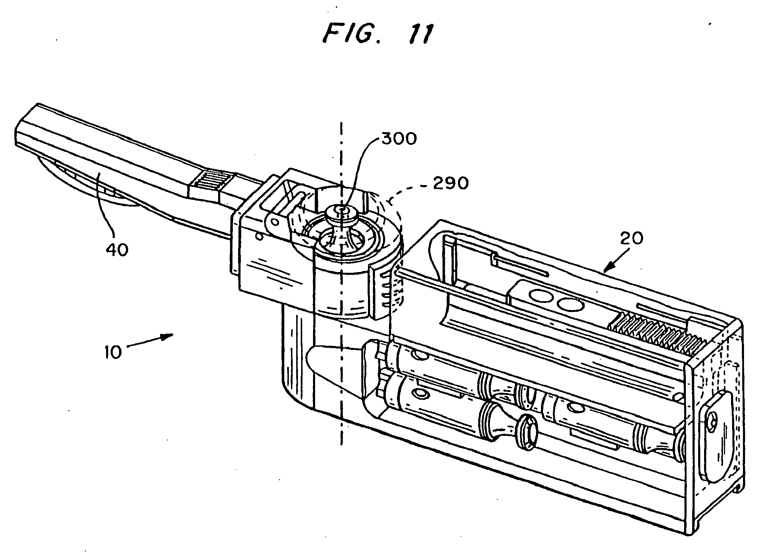

Figure 9, Figure 10, and Figure 11 show one specific example of the dry particle inhaler (10). In Figure 9, the cover (90) of the mouthpiece is closed, and several capsule (300) are in the storage section (470). In Figure 10, the mouthpiece (40) has been rotated relative to the intake section (20). The longitudinal axis (60) [not shown] of the intake section here makes an approximately ninety degree angle with the longitudinal axis (70) of the mouthpiece section. This permits the cover (290) for the mixing section to be opened. A medicament capsule (300) taken from the storage section (470) is about to be inserted into the mixing section (30). In Figure 11, the mouthpiece (40) has been rotated to a fully extended position, the cover (290) for the mixing section has been closed, and the dry particle inhaler 910) is ready for use. In one example of the dry particle inhaler (10), when the dry particle inhaler is in the closed position (Figure 9), the interior of the intake section (20) would be isolated from the outside air, but the mouthpiece (40) interior and the mixing section interior (291) would not be, permitting them to dry out after being exposed to the humid breath of a user.

Figure 12, Figure 13, Figure 14, and Figure 15 show a temporal sequence where a capsule (300) of medicament is loaded into the mixing section (30) of a dry particle inhaler (10), and the mouthpiece (40) is extended for use. The dry particle inhaler (10) described herein can also be used for nasal delivery of medicaments. A small tube (not shown) can be fitted to the end of the mouthpiece (40), and the other end of the tube inserted into the nostril. Alternatively, the mouthpiece (40) may be replaced by a nosepiece (not shown), whose free end is sized to be inserted into a nostril of a user. In another example, a device such as a bellows or a syringe is used to force air through the dry particle inhaler (10) into a nosepiece inserted into the nostril of a user (not shown).

Figure 16 shows the fluid (air) flow of the dry particle inhaler (10) modeled as the equivalent electrical circuit. This is styled a "pneumatic resistance circuits.

Figure 17 shows a schematic view of the dry particle inhaler (10). The air passage (50) through the dry particle inhaler widens as it goes through the mouthpiece (40) along the direction of the air flow (460). The opening (135) of the mouthpiece to be inserted into the mouth of the user may be roughly ellipsoid, or oval, and thus have a major axis and a minor axis. The ratio of these two may be called the horizontal aspect ratio. In one embodiment of the invention, the horizontal aspect ratio is between 2: 1 and 4: 1. In one embodiment of the dry particle inhaler (10), the horizontal aspect ratio is 3 : 1. Shaping the opening (135) in this manner keeps the drug particles collimated, maintains the optimal velocity of the particles in the air stream, and is oriented to the natural horizontal aspect ratio of the oropharyngeal region of the mouth. In one example , the outline of the opening (135) resembles a bean.

The dry particle inhaler described herein may be used with medicament particles of low, medium, and high shear forces.

The dry particle inhaler and capsules described herein may be made with a variety of suitable materials known to those skilled in the art, such as metal, glass, rubber, and plastic.

While the invention has been described with reference to particular embodiments, those skilled in the art will be able to make various modifications without departing from the scope thereof.

## Claims

1. A medicament capsule (300) for a dry powder inhaler, comprising a first tube (310) and a second tube (380), and a locking mechanism; wherein each tube is structurally configured to have a chamber with an open end (320; 390) and a closed end (330; 400), an inner surface, and an outer surface; wherein each tube comprises at least one secondary hole (370; 440) in its wall, and at least one keying surface (360; 430) that extends from the outer surface of one of the tubes; wherein the first tube (310) and second tube (380) fit together to form the capsule (300) and are rotatable relative to each other; and wherein the capsule has a cylindrical, rectangular or ovoid shape; **characterised in that** the at least one secondary hole (370) in the wall of the first tube (310) can be placed in alignment with the at least one secondary hole (440) in the wall of the second tube (330).

2. The medicament capsule of Claim 1, wherein the locking mechanism comprises protrusions (350; 420) on the surfaces of the first tube and second tube.

3. The medicament capsule of Claim 1, wherein the at least one keying surface is provided on one or both of the first tube (310) and the second tube (380) and structurally configured to mate with an inhaler.

4. The medicament capsule of Claim 1, wherein the at least one keying surface (360; 430) is provided on an end of one or both tubes.

5. The medicament capsule of Claim 1, wherein the keying surface (360) of the first tube (310) differs from the keying surface (430) of the second tube (380) so as to permit tactile and visual identification of the orientation of the capsule.

6. The medicament capsule of Claim 5, wherein the keying surfaces (360; 430) are configured in the shape of a rectangle, triangle, hexagon, letter Z or letter C.

7. The medicament capsule of Claim 1, wherein the first tube (310) is rotatable relative to the second tube (380) to cause the at least one secondary hole (370) in the first tube (310) and the at least one secondary hole (440) in the second tube (380) to adopt an aligned position; and optionally a non-aligned position.

8. The medicament capsule of Claim 7, wherein when the at least one secondary hole (370) in the first tube (310) and the at least one secondary hole (440) in the second tube (380) are in the aligned position release of a medicament in the chamber can occur when air passes in, through and out of the capsule (300).

9. The medicament capsule of Claim 1, wherein the intersection of the surface defining one or more of the secondary holes (370; 440) and the surface defining the interior of the capsule (300) meet in a chamfered, or bevelled, edge (372).

10. The medicament capsule of Claim 1, wherein the capsule (300) includes a mixing chamber comprising cyclone-creating structures (375, 445) which cause air flow within the capsule to be cyclonic, aiding in mixing the medicament particles with air when air flows through the chamber.

11. The medicament capsule of Claim 10, wherein the cyclone-creating structures (375, 445) are cones.

12. The medicament capsule of Claim 1, wherein the keying surface of the first tube is mateable with mechanical fasteners of a storage section of an inhalation device.

13. A capsule according to Claim 1 wherein the at least one keying surface (360; 430) aligns the capsule in the inhaler, and wherein said keying surface (360; 430) comprises a protrusion or a slot which enables identification of the capsule, or mates with a complementary keying surface in the inhaler.

14. The capsule of Claim 13, wherein the first tube (310) and the second tube (380) are retained one inside the other to define the capsule body, such that an end of each said tube defines respectively a top or bottom end of the capsule, the capsule further comprising structures in an interior surface causing in use cychronic flow in said capsule (300).

15. The capsule of Claim 14, further comprising a respective keying surface (360; 430) or fastening mechanism on an end of one or both said tubes.

16. The capsule of Claim 13, wherein the first tube (310) is inserted into the open end (390) of the second tube (380), wherein a protrusion (350) on the outer surface of the first tube (310) may slide past a protrusion (420) on the inner surface of the second tube (380), locking the first and the second tubes (310; 380) together; and wherein when the tubes (310; 380) are locked together at least one opening (370) in the first tube (310) may be made coincident with a least one opening (440) in the second tube (380), by rotation of the first (310) and second (380) tubes one inside the other.

## Patentansprüche

1. Eine Medikamcntenkapsel (300) für einen Trockenpulverinhalator, mit einem ersten Rohr (310) und einem zweiten Rohr (380) und einem Verriegelungsmechanismus, wobei jedes Rohr strukturell mit einer Kammer mit einem offenen Ende (320;390) und einem geschlossenen Ende (330;400), einer Innenfläche und einer Außenfläche konf-guriert ist, wobei jedes Rohr mindestens ein Sekundärloch (370;440) in seiner Wand und mindestens eine Passfläche (360;430) aufweist, welche sich von der Außenfläche eines der Rohre erstreckt, wobei das erste Rohr (310) und das zweite Rohr (380) zusammenpassen, um die Kapsel (300) zu bilden, und relativ zueinander drehbar sind, und wobei die Kapsel eine zylindrische, eine rechteckige oder eine ovale Form besitzt, dadurch gekenntzeichnet, dass das mindestens eine Sekundärloch (370) in der Wand des ersten Rohrs (310) in Ausrichtung mit dem mindestens einen Sekundärloch (440) in der Wand des zweiten Rohrs (330) angeordnet werden kann.

2. Die Medikamentenkapsel gemäß Anspruch 1, wobei der Verriegelungsmechanismus Vorsprünge (350;420) an den Oberflächen des ersten Rohrs und des zweiten Rohrs aufweist.

3. Die Medikamentenkapsel gemäß Anspruch 1, wobei die mindestens eine Passfläche an einem oder an beiden von erstem Rohr (310) und zweitem Rohr (380) vorgesehen und strukturell konfiguriert ist, um mit einem Inhalator zusammenzupassen.

4. Die Medikamentenkapsel gemäß Anspruch 1, wobei die mindestens eine Passfläche (360;430) an einem Ende von einem oder beiden Rohren vorgesehen ist.

5. Die Medikamentenkapsel gemäß Anspruch 1, wobei die Passfläche (360) des ersten Rohrs (310) sich von der Passfläche (430) des zweiten Rohrs (380) unterscheidet, um eine taktile und visuelle Identifikation der Orientierung der Kapsel zu ermöglichen.

6. Die Medikamentenkapsel gemäß Anspruch 5, wobei die Passflächen (360;430) in der Form eines Rechtecks, eines Dreiecks, eines Hexagons, eines Buchstabens Z oder eines Buchstabens C konfiguriert sind.

7. Die Medikamentenkapsel gemäß Anspruch 1, wobei das erste Rohr (310) relativ zu dem zweiten Rohr (380) drehbar ist, um das mindestens eine Sekundärloch (370) in dem ersten Rohr (310) und das mindestens eine Sekundärloch (440) in dem zweiten Rohr (380) eine ausgerichtete Position und optional eine nicht-ausgerichtete Position annehmen zu lassen.

8. Die Medikamentenkapsel gemäß Anspruch 7, wobei, wenn das mindestens eine Sekundärloch (370) in dem ersten Rohr (310) und das mindestens eine Sekundärloch (440) in dem zweiten Rohr (380) in der ausgerichteten Position sind, Freisetzung eines Medikaments in der Kammer auftreten kann, wenn Luft in, durch und aus der Kapsel (300) tritt.

9. Die Medikamentenkapsel gemäß Anspruch 1, wobei der Schnitt der Oberflache, die eines oder mehrere der Sekundärlöcher (370;440) definiert, mit der Oberfläche, die das Innere der Kapsel (300) definiert, in einem abgeschrägten bzw. angefasten oder einer konischen bzw. schrägverlaufenden Rand (372) zusammentrifft.

10. Die Medikamentenkapsel gemäß Anspruch 1, wobei die Kapsel (300) eine Mischkammer mit Zyklon-erzeugenden Strukturen (375,445) umfasst, welche einen Luftstrom in der Kapsel zu einem Zyklon machen, was beim Mischen der Medikamententeilchen mit Luft hilft, wenn Luft durch die Kammer strömt.

11. Die Medikamentenkapsel gemäß Anspruch 10, wobei die Zyklon-erzeugenden Strukturen (375,445) Kegel bzw. Zapfen sind.

12. Die Medikamentenkapsel gemäß Anspruch 1, wobei die Passfläche des ersten Rohrs mit mechanischen Befestigern eines Speicherabschnitts einer Inhalationsvorrichtung zusammensetzbar ist.

13. Eine Kapsel gemäß Anspruch 1, wobei die mindestens eine Passfläche (360;430) die Kapsel in dem Inhalator ausrichtet, und wobei die Passfläche (360;430) einen Vorsprung oder einen Schlitz aufweist, der eine Identifikation der Kapsel ermöglicht, oder mit einer komplementären Passfläche in dem Inhalator zusammenpasst.

14. Kapsel gemäß Anspruch 13, wobei das erste Rohr (310) und das zweite Rohr (380) gegenseitig ineinander gehalten sind, um den Kapselkörper zu definieren, derart, dass ein Ende jedes Rohrs jeweils ein oberes oder unteres Ende der Kapsel definiert, wobei die Kapsel ferner Strukturen in einer Innenfläche besitzt, welche im Einsatz eine zyklonische Strömung in der Kapsel (300) bewirken.

15. Die Kapsel gemäß Anspruch 14, ferner mit einer jeweiligen Passfläche (36C;430) oder einem Befestigungsmechanismus an einem Ende von einem oder beiden Rohren.

16. Die Kapsel gemäß Anspruch 13, wobei das erste Rohr (310) in das offene Ende (390) des zweiten Rohrs (380) eingesetzt ist, wobei ein Vorsprung (350) an der Außenfläche des ersten Rohrs (310) über einen Vorsprung (420) an der Innenfläche des zweiten Rohrs (380) hinaus gleiten kann, wodurch das erste und das zweite Rohr (310;380) zusammen verriegelt werden, und wobei, wenn die Rohre (310;380) zusammen verriegelt sind, mindestens eine Öffnung (370) in dem ersten Rohr (310) in Überstimmung mit mindestens einer Öffnung (440) in dem zweiten Rohr (380) zur Übereinstimmung gebracht werden kann, durch Drehung des ersten (310) und zweiten (380) Rohrs ineinander.

## Revendications

1. Capsule de médicament (300) pour un inhalateur à poudre sèche, comprenant un premier tube (310) et un second tube (380), et un mécanisme de verrouillage ; dans laquelle chaque tube est structurellement configuré pour avoir une chambre avec une extrémité ouverte (320 ; 390) et une extrémité fermée (330 ; 400), une surface interne et une surface externe ; dans laquelle chaque tube comprend au moins un trou secondaire (370 ; 440) dans sa paroi, et au moins une surface de manipulation (360 ; 430) qui s'étend depuis la surface externe de l'un des tubes ; dans laquelle le premier tube (310) et le second tube (380) s'assemblent pour former la capsule (300) et peuvent tourner l'un par rapport à l' autre ; et dans laquelle la capsule a une forme cylindrique, rectangulaire ou ovoïde ; **caractérisée en ce que** le au moins un trou secondaire (370) dans la paroi du premier tube (310) peut être placé en alignement avec le au moins un trou secondaire (440) dans la paroi du second tube (330).

2. Capsule de médicament selon la revendication 1, dans laquelle le mécanisme de verrouillage comprend des saillies (350 ; 420) sur les surfaces du premier tube et du second tube.

3. Capsule de médicament selon la revendication 1, dans laquelle la au moins une surface de manipulation est disposée sur le premier tube (310) et/ou le second tube (380) et structurellement configurée pour s'apparier avec un inhalateur.

4. Capsule de médicament selon la revendication 1, dans laquelle la au moins une surface de manipulation (360 ; 430) est disposée sur une extrémité d'un ou des deux tubes.

5. Capsule de médicament selon la revendication 1, dans laquelle la surface de manipulation (360) du premier tube (310) diffère de la surface de manipulation (430) du second tube (380) de façon à permettre une identification tactile et visuelle de l'orientation de la capsule.

6. Capsule de médicament selon la revendication 5, dans laquelle les surfaces de manipulation (360 ; 430) sont configurées sous la forme d'un rectangle, d'un triangle, d'un hexagone, de la lettre Z ou de la lettre C.

7. Capsule de médicament selon la revendication 1, dans laquelle le premier tube (310) peut tourner par rapport au second tube (380) pour amener le au moins un trou secondaire (370) dans le premier tube (370) et le au moins un trou secondaire (440) dans le second tube (380) à adopter une position alignée ; et facultativement une position non alignée.

8. Capsule de médicament selon la revendication 7, dans laquelle lorsque le au moins un trou secondaire (370) dans le premier tube (310) et le au moins un trou seconda-re (440) dans le second tube (380) sont dans la position alignée, la libération d'un médicament dans la chambre peut se produire lorsque de l'air entre, passe à travers et sort de la capsule (300).

9. Capsule de médicament selon la revendication 1, dans laquelle l'intersection de la surface définissant un ou plusieurs des trous secondaires (370 ; 440) et la surface définissant l'intérieur de la capsule (300) se coupe en un bord chanfreiné ou biseauté (372).

10. Capsule de médicament selon la revendication 1, dans laquelle la capsule (300) inclut une chambre de mélange comprenant des structures de formation de cyclone (375, 445) qui rendent le flux d'air à l'intérieur de la capsule cyclonique, ce qui aide au mélange des particules de médicament avec l'air lorsque de l'air circule dans la chambre.

11. Capsule de médicament selon la revendication 10, dans laquelle les structures de formation de cyclone (375, 445) sont des cônes.

12. Capsule de médicament selon la revendication 1, dans laquelle la surface de manipulation du premier tube peut s'apparier avec des fixations mécaniques d'une partie de stockage d'un dispositif d'inhalation.

13. Capsule selon la revendication 1, dans laquelle la au moins une surface de manipulation (360 ; 430) aligne la capsule dans l'inhalateur, et dans laquelle ladite surface de manipulation (360 ; 430) comprend une saillie ou une fente qui permet l'identification de la capsule, ou s'apparie avec une surface de manipulation complémentaire dans l'inhalateur.

14. Capsule selon la revendication 13, dans laquelle le premier tube (310) et le second tube (380) sont retenus l'un à l'intérieur de l'autre pour définir le corps de la capsule, de telle manière qu'une extrémité de chacun desdits tubes définit respectivement une extrémité supérieure ou inférieure de la capsule, la capsule comprenant en outre des structures dans une surface intérieure rendant durant l'utilisation le flux cyclonique dans ladite capsule (300).

15. Capsule selon la revendication 14, comprenant en outre une surface de manipulation (360, 430) respective ou un mécanisme de fixation respectif sur une extrémité de l'un desdits tubes ou des deux.

16. Capsule selon la revendication 13, dans laquelle le premier tube (310) est inséré dans l'extrémité ouverte (390) du second tube (380), dans laquelle une saillie (350) sur la surface externe du premier tube (310) peut coulisser devant une saillie (420) sur la surface interne du second tube (380), ce qui verrouille les premier et second tubes (310 ; 380) l'un avec l'autre ; et dans laquelle lorsque les tubes (310 ; 380) sont verrouillés l'un avec l'autre, il est possible de faire coïncider au moins une ouverture (370) dans le premier tube (310) avec au moins une ouverture (440) dans le second tube (380), par rotation des premier (310) et second (380) tubes l'un à l'intérieur de l'autre.
